# FASCICULE DE BREVET EUROPEEN

(11) **EP 0 505 238 B1**
(45) Date de publication et mention de la délivrance du brevet: **03.01.1996**
(21) Numéro de dépôt: 92400639.8
(22) Date de dépôt: 11.03.1992
(51) Int. Cl.: C07C 7/08

(54) **Procédé de séparation de butènes et de butanes par distillation extractive**
Verfahren zur Trennung von Butenen und Butanen durch Extractive Destillation
Process for the separation of butenes and butanes by extractive distillation

(30) Priorité: 20.03.1991 FR 9103477
(43) Date de publication de la demande: 23.09.1992
(73) Titulaire: INSTITUT FRANCAIS DU PETROLE, F-92502 Rueil-Malmaison (FR)
(72) Inventeur: Asselineau, Lionel, F-75017 Paris (FR); Rojey, Alexandre, F-92500 Rueil Malmaison (FR)

(56) Documents cités:
- EP-A- 0 079 679
- FR-A- 2 016 342
- FR-A- 2 520 356

## Description

L'invention concerne un procédé de séparation des butanes et des butènes par distillation extractive.

Dans le traitement de la coupe C₄ issue du vapocraquage ou du craquage catalytique, la séparation des butènes-butanes se situe généralement en aval de la distillation extractive du butadiène-1,3 et de l'unité de synthèse du méthyl tertiobutyl éther (MTBE). On dispose donc d'une coupe C₄ débarrassée du butadiène-1,3 et très appauvrie en isobutène.

Les butènes étant des produits valorisables, on est amené à séparer les butènes des butanes. En effet, les butènes peuvent être soit eux-mêmes séparés en butène-1 et butènes-2, soit isomérisés en isobutène, qui est recyclé dans l'unité de synthèse de MTBE. Les butènes peuvent être également dimérisés par le procédé DIMERSOL (marque déposée) en produits utilisables dans les essences. Cette dimérisation est un procédé réalisé par catalyse homogène, dans lequel le catalyseur est perdu. L'efficacité du catalyseur étant proportionnelle à sa concentration dans la charge, une charge débarrassée de paraffines permet une économie de catalyseur. De plus, les butènes qui n'ont pas réagi peuvent être recyclés si la charge a été préalablement débarrassée des butanes.

Il est connu de séparer les butanes et les butènes dans une coupe C₄ par distillation extractive. Un procédé décrit antérieurement (S. OGURA, T. ONDA, Advances in C₄ Hydrocarbon Processing AlChE 1987 Summer National Meeting, August 16-19, 1987) consiste à réaliser la distillation extractive avec comme solvant le diméthylformamide (DMF) sous pression, la récupération du solvant étant effectuée sous pression atmosphérique et la purification des butènes étant effectuée sous pression. Le produit de fond de la colonne de distillation extractive est envoyé dans une colonne sous pression atmosphérique, dans laquelle les butènes sont désorbés et sortent en tête avec une partie du solvant, tandis que le produit de fond est constitué de DMF pratiquement pur. Les butènes de tête sont ensuite envoyés, pour être purifiés, dans une colonne sous pression. Le solvant récupéré en fond de cette dernière colonne et qui contient encore des butènes, retourne dans la colonne atmosphérique.

L'inconvénient d'opérer suivant ce procédé est que la majorité des butènes de la charge doit être distillée deux fois, ce qui, compte tenu de la recompression nécessaire des vapeurs de butènes, entraîne une consommation énergétique élevée.

Dans une demande de brevet antérieure FR-A-2 673 178 à laquelle correspond la demande européenne EP-A-501 848 le déposant a déjà décrit un procédé de séparation des butènes et des butanes dans lequel la charge comprenant les butanes et les butènes à est introduite dans une colonne de distillation extractive sous pression, où elle est mise en contact avec un solvant polaire dans lequel les butanes ont une volatilité plus élevée que les butènes, le distillat sortant en tête consistant essentiellement en les butanes séparés ; le résidu recueilli en fond, comprenant principalement le solvant et les butènes, est envoyé dans une colonne sous pression dans laquelle la température de fond est ajustée de manière que la désorption des butènes ne soit pas complète, ce qui permet de limiter la température de fond de colonne et par conséquent d'éviter la décomposition thermique du solvant.

Le solvant qui sort en fond, contenant une fraction des butènes, est envoyé dans une colonne de purification sous une pression voisine de la pression atmosphérique dont la marche est réglée de manière que la vapeur de tête, constituée principalement de butènes, contienne aussi une fraction du solvant, ce qui permet de la condenser partiellement pour assurer le reflux de la colonne, en évitant la compression à ce stade. Le distillat vapeur est envoyé, après compression, dans la colonne de désorption sous pression ; le solvant purifié sortant en fond est recyclé vers la colonne de distillation extractive.

On a maintenant découvert qu'il était possible de modifier le procédé ainsi décrit, en faisant jouer à un second solvant convenablement choisi le rôle joué par la fraction des butènes qui était maintenue en mélange avec le solvant polaire dans le résidu de la colonne de désorption (c'est-à-dire d'abaisser la température d'ébullition de ce résidu de manière à éviter l'altération thermique du solvant). La mise en jeu d'un second solvant permet de s'affranchir de l'étape de compression de l'effluent vapeur de la colonne de purification qui, constitué principalement de butènes, devait être recyclé vers la colonne de désorption sous pression.

Le procédé de l'invention présente donc une simplicité de mise en oeuvre accrue.

Le procédé de séparation des butènes et des butanes de la présente invention est défini en général d'une manière analogue au procédé de la demande antérieure, avec certaines modifications tenant à la mise en jeu d'un solvant secondaire.

Comme dans la demande antérieure, la charge à traiter est en général une coupe C₄ de vapocraquage ou de craquage catalytique qui a été débarrassée du butadiène-1,3, par exemple par distillation extractive, et dont la teneur en isobutène peut avoir été réduite, par exemple dans une unité de synthèse du MTBE.

Les charges considérées comprennent donc en général principalement des butènes (butène-1, butène-2 cis et butène-2 trans), du n-butane, de l'isobutane, de l'isobutène en faible proportion et, à l'état de traces, des hydrocarbures à 3 et 5 atomes de carbone.

Une composition plus particulière de la charge peut être par exemple :
- Isobutane: de 15 à 20 % en poids
- N-butane: de 7 à 15 % en poids
- Butène-1: de 20 à 25 % en poids
- Isobutène: <5 % en poids
- Butène-2 trans: de 25 à 30 % en poids
- Butène-2 cis: de 15 à 20 % en poids

Le solvant utilisé comme premier solvant dans le procédé de la présente invention (désigné par S₁) peut être, comme dans la demande antérieure, tout solvant polaire sélectif, c'est-à-dire dans lequel les butanes ont une volatilité plus élevée que les butènes. A titre d'exemples non limitatifs, on peut citer le monométhylformamide (Eb = 182 °C), le diméthylformamide (Eb = 153 °C), le diéthylformamide (Eb = 177,5 °C), le diméthylacétamide (Eb = 165 °C) et la N-méthylpyrrolidone (Eb = 202 °C). On utilise le plus souvent le diméthylformamide.

Comme second solvant (désigné par S₂), on utilise dans le procédé de la présente invention tout composé organique condensable sous pression atmosphérique ayant une volatilité intermédiaire entre celle du premier solvant et celle des butènes, ne formant pas d'azéotrope avec les butènes et miscible avec le premier solvant. Il doit en effet être assez volatil pour que la température d'ébullition du mélange qu'il forme en fond de colonne de désorption avec le premier solvant soit suffisamment abaissée, mais ne doit pas être trop volatil pour que sa condensation en sortie de la colonne de purification reste assez aisée. Une température d'ébullition dans l'intervalle de 30 à 80 °C apparaît comme convenable pour réaliser les buts de l'invention. A titre d'exemples non limitatifs, on peut citer l'hexane (Eb = 68 °C), le cyclohexane (Eb = 80 °C), le benzène (Eb = 80 °C), le méthyl tertiobutyl éther (Eb = 55 °C), des mélanges de ces solvants ainsi que des coupes d'hydrocarbures en C₅ ou C₆ (Eb entre 30 et 70 °C).

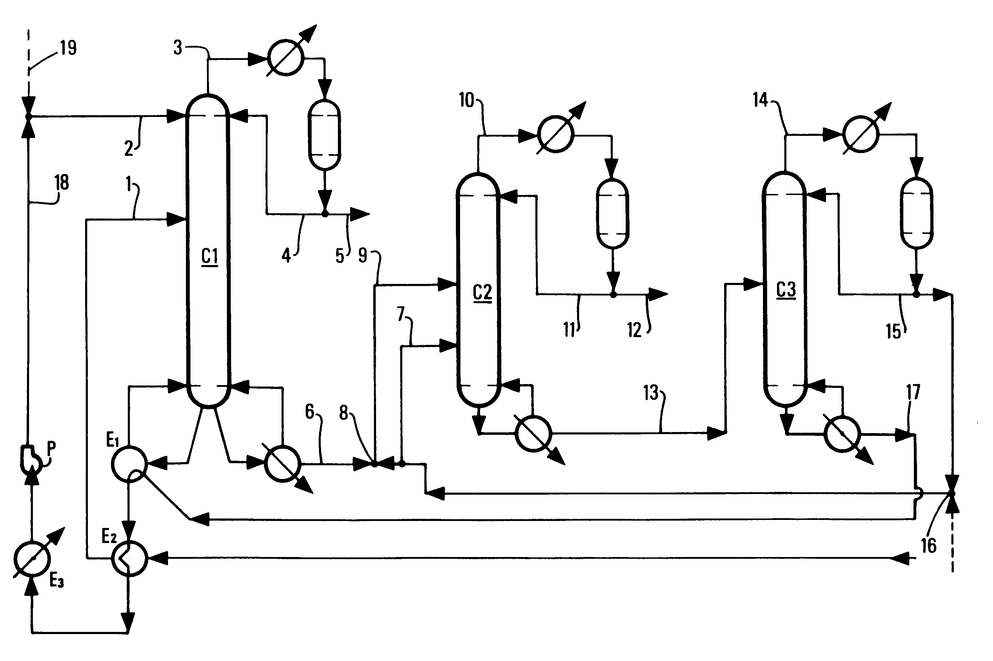

Le procédé de l'invention sera décrit ci-après de façon plus détaillée, en liaison avec la figure annexée, qui représente un agencement simplifié pour sa mise en oeuvre.

La charge à traiter, préalablement réchauffée par exemple à une température de 50 à 70 °C à travers l'échangeur E₂, est introduite par la ligne 1 dans la colonne de distillation extractive C₁ sous une pression p₁ de 4 à 10 bars.

Le solvant d'extraction, à une température inférieure à sa température d'ébullition à la pression considérée, est introduit, par la ligne 2, dans la partie supérieure de la colonne C₁. La température d'introduction du solvant est par exemple de 50 à 90 °C. Le débit de solvant peut être avec le débit de charge dans un rapport pondéral de 3 à 15 kg/kg.

La colonne C₁ travaille par exemple avec une température de fond de 90 à 140 °C. La température de tête s'établit à une valeur de 30 à 70 °C.

Le distillat sortant en tête par la ligne 3 comprend principalement l'isobutane et le n-butane, et éventuellement un peu de butène-1 et d'isobutène, en fonction du réglage de la colonne. Une fraction du distillat, après condensation, est renvoyée, à titre de reflux liquide, vers la colonne C₁, par la ligne 4. On recueille le reste de l'effluent par la ligne 5, qui peut par exemple être renvoyé au pool C₄.

Le résidu sortant en fond de colonne C₁ par la ligne 6, ne contenant pratiquement plus de butanes est envoyé dans la colonne de désorption C₂ opérant à une pression p₂ voisine de la pression p₁. On introduit également dans la colonne C₂, par la ligne 7, un flux de solvant secondaire S₂, en une quantité telle que l'on puisse recueillir les butènes en tête de la colonne C₂ à une température de 40 à 60 °C à la pression p₂, tout en maintenant la température de fond, par exemple de 150 à 170 °C, inférieure aux températures auxquelles le solvant S₁ commence a s'altérer de façon significative. La quantité de solvant S₂ est par exemple de 15 à 50 % en poids de celle de S₁.

Le solvant S₂ peut éventuellement être mélangé en 8 au résidu de colonne C₁, le mélange étant alors introduit dans la colonne C₂ par la ligne 9.

Le distillat sortant par la ligne 10, constitué des butènes séparés avec une pureté par exemple d'au moins 97 % en poids, est condensé et renvoyé en partie, à titre de reflux, vers la colonne C₂ par la ligne 11. On recueille le reste par la ligne 12.

Le résidu de la colonne C₂, sortant par la ligne 13 et consistant essentiellement en un mélange des solvants S₁ et S₂ est envoyé dans la colonne de purification C₃ travaillant à une pression p₃ d'environ 1 bar, par exemple de 1 à 3 bars, et entre une température de fond de 140 à 170 °C et une température de tête de 40 à 60 °C.

Le distillat sortant en tête de la colonne C₃ par la ligne 14 consiste essentiellement en le solvant S₂ et peut contenir une faible proportion de solvant S₁. Il est condensé et renvoyé en partie à titre de reflux vers la colonne C₃ par la ligne 15. L'autre partie, après un éventuel appoint en 16, est envoyée en alimentation de la colonne C₂ par la ligne 7, ou remélangée en 8 avec le fond de la colonne de distillation extractive C₁, pour constituer le mélange alimentant par la ligne 9 la colonne de désorption C₂.

Le résidu de fond de colonne C₃, sortant par la ligne 17 est du solvant S₁ pratiquement pur. Il est recyclé vers la colonne de distillation extractive C₁. L'énergie calorifique transportée par ce flux de solvant S₁, en général à une température de 150 à 170 °C, peut être utilisée en partie pour chauffer le fond de la colonne C₁ par l'intermédiaire de l'échangeur E₁ et en partie pour réchauffer la charge jusqu'à son point de bulle par l'intermédiaire de l'échangeur E₂. Le flux de solvant S₁ peut encore être refroidi à travers l'échangeur E₃ avant d'être envoyé par la pompe P et la ligne 18 dans la colonne C₁. Un appoint de solvant peut être effectué par la ligne 19.

Par le procédé de l'invention, on peut obtenir les butènes avec un degré de pureté par exemple d'environ 97 % en poids ou davantage si nécessaire, principalement selon la proportion de solvant S₁ utilisé et l'efficacité (nombre de plateaux) de la colonne de distillation extractive.

Dans la présente description, les pressions indiquées sont des pressions absolues. On rappelle que 1 bar vaut 0,1 MPa.

On donne ci-après un exemple de réalisation pratique du procédé de l'invention.

### EXEMPLE

La charge à traiter est un mélange d'hydrocarbures dont la composition est donnée dans la deuxième colonne du tableau.

Le solvant S₁ utilisé est le diméthylformamide (DMF). Le solvant S₂ est l'hexane.

Les colonnes utilisées présentent les caractéristiques suivantes :

La colonne de distillation extractive C₁ est une colonne en acier de 50 mm de diamètre qui comporte 100 plateaux perforés à déversoir.

La colonne de désorption C₂ est une colonne en acier de 50 mm de diamètre qui comporte 65 plateaux perforés à déversoir.

La colonne de purification C₃ est une colonne en verre de 50 mm de diamètre qui comporte 20 plateaux.

Chacune de ces colonnes est rendue adiabatique, par compensation thermique pour les colonnes en acier, par isolation thermique pour la colonne en verre.

La colonne C₁ est alimentée au niveau du 62e plateau (les plateaux sont comptés de haut en bas), par 435 g/h de charge, à une température de 52 ° C.

Au niveau du 10e plateau, elle est alimentée par le solvant, le diméthylformamide, à une température de 65 °C et un débit de 2770 g/h.

La pression est établie à 5,6 bars, le taux de reflux à 7,25, et la colonne régulée pour délivrer 129 g/h de distillat. La composition du distillat produit est présentée dans la troisième colonne du tableau.

La colonne C₂ est alimentée par le mélange de 3076 g/h de résidu de la colonne C₁ et de 947 g/h de distillat condensé de la colonne C₃, dont la composition est donnée dans la cinquième colonne du tableau.

La pression est établie à 5 bars, le taux de reflux à 1,1 et la colonne est régulée pour délivrer 307 g/h de distillat.

La composition du distillat produit, constitué des butènes purifiés, est donnée dans la quatrième colonne du tableau.

La colonne C₃ est alimentée au niveau du huitième plateau par le résidu de la colonne C₂, à raison de 3716 g/h.

Elle fonctionne sous pression atmosphérique, avec un taux de reflux égal à 1. Le distillat est soutiré avec un débit de 947 g/h et est recyclé à la colonne C₂ comme décrit précédemment. Le résidu, constitué de DMF quasi pur est recyclé à la colonne C₁.

| | Charge C₁ | Distillat C₁ | Distillat C₂ | Distillat C₃ |
|---|---|---|---|---|
| Débit g/h | 435 | 129 | 307 | 947 |

| Composition % poids | | | | |
|---|---|---|---|---|
| Isobutane | 18,4 | 62,2 | 0,02 | - |
| N-butane | 10,5 | 30,0 | 2,25 | - |
| Butène-1 | 23,3 | 7,35 | 30,0 | - |
| Isobutène | 1,5 | 0,38 | 2,03 | - |
| Butène-2 trans | 27,9 | 0,06 | 39,6 | - |
| Butène-2 cis | 18,4 | <0,01 | 26,1 | - |
| DMF | - | - | - | 4,36 |
| Hexane | - | - | - | 95,64 |

## Revendications

1. Procédé de séparation de butanes et de butènes à partir d'une charge en contenant, dans lequel ladite charge est introduite dans une colonne de distillation extractive sous pression, dans laquelle elle est mise en contact avec un premier solvant S₁, polaire, dans lequel les butanes ont une volatilité plus élevée que les butènes, le distillat sortant en tête consistant essentiellement en les butanes, ledit procédé étant caractérisé en ce que le résidu recueilli en fond de ladite colonne de distillation extractive, comprenant principalement le solvant S1 et les butènes, est envoyé conjointement à un second solvant S2 ayant une volatilité intermédiaire entre celle dudit premier solvant S1 et celle des butènes, ne formant pas d'azéotrope avec les butènes et miscible avec ledit premier solvant S₁, dans une colonne de désorption sous pression, le distillat sortant en tête étant constitué essentiellement des butènes, le résidu consistant en un mélange des solvants S₁ et S₂, est envoyé dans une colonne de séparation sous une pression voisine de la pression atmosphérique, de laquelle le distillat sortant en tête consiste essentiellement en ledit solvant S₂, qui est recyclé vers la colonne de désorption et le résidu de fond consiste en ledit solvant S1 pratiquement pur, qui est recyclé vers la colonne de distillation extractive.

2. Procédé selon la revendication 1, caractérisé en ce que ledit premier solvant polaire S₁ est choisi parmi le monométhylformamide, le diméthylformamide, le diéthylformamide, le diméthylacétamide et la N-méthylpyrrolidone.

3. Procédé selon l'une des revendications 1 et 2, caractérisé en ce que ledit second solvant S2 a une température d'ébulliton de 30 à 80 °C.

4. Procédé selon l'une des revendications 1 à 3, caractérisé en ce que ledit second solvant S₂ est choisi parmi l'hexane, le cyclohexane, le benzène, le méthyl tertiobutyl éther et les coupes d'hydrocarbures en C₅ ou C₆.

5. Procédé selon l'une des revendications 1 à 4, caractérisé en ce que la colonne de distillation extractive fonctionne sous une pression de 4 à 10 bars ; le solvant S₁ est introduit dans la partie supérieure de ladite colonne de distillation extractive sous un débit dans un rapport pondéral de 3 à 15 avec le débit de ladite charge ; la température de fond de colonne est de 90 à 140 °C et la température de tête de 30 à 70 °C.

6. Procédé selon l'une des revendications 1 à 5, caractérisé en ce que le solvant S₂ est introduit dans la colonne de désorption sous un débit de 15 à 50 % par rapport au débit de solvant S₁, la pression de ladite colonne de désorption est de 4 à 10 bars, la température de fond est de 150 à 170 °C et la température de tête de 40 à 60 °C.

7. Procédé selon l'une des revendications 1 à 6, caractérisé en ce que la pression de la colonne de purification est d'environ 1 bar, la température de fond est de 140 à 170°C et la température de tête de 30 à 100°C.

8. Procédé selon l'une des revendications 1 à 7, caractérisé en ce que l'on obtient un mélange de butènes ayant une pureté d'au moins 97 % en poids.

## Claims

1. Process for the separation of butanes and butenes from a charge containing the same, in which said charge is introduced into an extractive distillation column under pressure, in which it is contacted with a first polar solvent S1, in which the butanes have a higher volatility than the butenes, the distillate passing out at the top consisting essentially of butanes, the process being characterized in that the residue collected at the bottom of said extractive distillation column and consisting mainly of solvent S1 and butenes is supplied jointly with a second solvent S2 having a volatility intermediate between that of the first solvent S1 and that of the butenes, which does not form an azeotrope with the butenes and which is miscible with said first solvent S1 into a desorption column under pressure, the distillate passing out at the head being constituted essentially by butenes, the residue consisting of a mixture of solvents S1 and S2 being fed into a separation column under a pressure close to atmospheric pressure, from which the distillate leaving at the head essentially consists of said solvent S2 and is recycled to the desorption column and the bottom residue consists of said substantially pure solvent S1 and is recycled to the extractive distillation column.

2. Process according to claim 1, characterized in that the first polar solvent S1 is chosen from among monomethyl formamide, dimethyl formamide, diethyl formamide, dimethyl acetamide and N-methyl pyrrolidone.

3. Process according to either of the claims 1 and 2, characterized in that the second solvent S2 has a boiling point of 30 to 80°C.

4. Process according to one of the claims 1 to 3, characterized in that the second solvent S2 is chosen from among hexane, cyclohexane, benzene, methyl tert. butyl ether and C₅ or C₆ hydrocarbon fractions.

5. Process according to one of the claims 1 to 4, characterized in that the extractive distillation column operates under a pressure of 4 to 10 bars, the solvent S1 being introduced into the upper part of the extractive distillation column at a flow rate in a weight ratio of 3 to 15 with the flow rate of said charge, the column bottom temperature being 90 to 140°C and the top temperature 30 to 70°C.

6. Process according to one of the claims 1 to 5, characterized in that the solvent S2 is introduced into the desorption column at a flow rate of 15 to 50% compared with that of the solvent S1, the pressure of said desorption column being 4 to 10 bars, the bottom temperature 150 to 170°C and the top temperature 40 to 60°C.

7. Process according to one of the claims 1 to 6, characterized in that the pressure of the purification column is approximately 1 bar, the bottom temperature is 140 to 170°C and the top temperature 30 to 100°C.

8. Process according to one of the claims 1 to 7, characterized in that a mixture of butenes is obtained with a purity of at least 97% by weight.

## Patentansprüche

1. Verfahren zum Trennen von Butanen und Butenen aus einer sie enthaltenden Charge, bei dem diese Charge in eine Extraktivdestillationskolonne unter Druck eingeführt wird, in der sie mit einem ersten polaren Lösungsmittel S₁ kontaktiert wird, in welchem die Butane eine höhere Flüchtigkeit als die Butene haben, wobei das am Kopf austretende Destillat im wesentlichen aus den Butanen besteht, dadurch gekennzeichnet, daß der am Boden der Extraktivdestillationskolonne gesammelte Rückstand, der hauptsächlich das Lösungsmittel S₁ und die Butene umfaßt, gemeinsam zu einem zweiten Lösungsmittel S₂, das eine Flüchtigkeit zwischen der des ersten Lösungsmittels S₁ und der der Butane hat, geschicht wird, wobei kein Azeotrop mit den Butenen gebildet wird und wobei die Mischbarkeit mit diesem Lösungsmittel S₁ gegeben ist, in einer Desorptionskolonne unter Druck, wobei das am Kopf austretende Destillat im wesentlichen aus den Butenen gebildet ist und der aus einem Gemisch der Lösungsmittel S₁ und S₂ bestehende Rückstand in eine Trennkolonne unter einem Druck benachbart dem atmosphärischen Druck geschicht wird, aus der das am Kopf austretende Destillat im wesentlichen aus diesem Lösungsmittel S₂ besteht, das zur Desorptionskolonne rezykliert wird, und der Bodenrückstand aus diesem praktisch reinen Lösungsmittel S₁ besteht, das zur Extraktivdestillationskolonne rezykliert wird.

2. Verfahren nach Anspruch 1, dadurch gekennzeichnet, daß dieses erste polare Lösungsmittel S₁ gewählt ist aus Monomethylformamid, Dimethylformamid, Diethylformamid, Dimethylacetamid und N-Methylpyrrolidon.

3. Verfahren nach einem der Ansprüche 1 und 2, dadurch gekennzeichnet, daß dieses zweite Lösungsmittel S₂ eine Siedetemperatur von 30 bis 80°C hat.

4. Verfahren nach einem der Ansprüche 1 bis 3, dadurch gekennzeichnet, daß dieses zweite Lösungsmittel S₂ gewählt ist aus Hexan, Cyclohexan, Benzol, Methyl-tert.-butylether und den Kohlenwasserstoffen-C₅- oder -C₆-Fraktionen.

5. Verfahren nach einem der Ansprüche 1 bis 4, dadurch gekennzeichnet, daß die Extraktivdestillationskolonne unter einem Druck von 4 bis 10 bar arbeitet; das Lösungsmittel in den oberen Teil der Extraktivdestillationskolonne mit einem Durchsatz bei einem Gewichtsverhältnis von 3 bis 15 zum Durchsatz dieser Charge eingeführt wird; und die Bodentemperatur der Kolonne bei 90 bis 140°C und die Kopftemperatur bei 30 bis 70°C liegt.

6. Verfahren nach einem der Ansprüche 1 bis 5, dadurch gekennzeichnet, daß das Lösungsmittel S₂ in die Desoptionskolonne mit einem Durchsatz von 15 bis 50 % bezogen auf den Durchsatz des Lösungsmittels S₁ eingeführt wird, wobei der Druck dieser Desorptionskolonne bei 4 bis 10 bar, die Bodentemperatur bei 150 bis 170°C und die Kopftemperatur bei 40 bis 60°C liegt.

7. Verfahren nach einem der Ansprüche 1 bis 6, dadurch gekennzeichnet, daß der Druck der Reinigungskolonne bei etwa 1 bar, die Bodentemperatur bei 140 bis 170°C und die Kopftemperatur bei 30 bis 100°C liegt.

8. Verfahren nach einem der Ansprüche 1 bis 7, dadurch gekennzeichnet, daß man ein Gemisch aus Butenen mit einer Reinheit von wenigstens 97 Gew.-% erhält.
